(19) 

Europäisches Patentamt
European Patent Office
Office européen des brevets

(11) **EP 3 542 722 B1**

(12) **EUROPEAN PATENT SPECIFICATION**

(45) Date of publication and mention
of the grant of the patent:
**21.05.2025 Bulletin 2025/21**

(21) Application number: **18728916.0**

(22) Date of filing: **15.03.2018**

(51) International Patent Classification (IPC):
*A61B 6/03* (2006.01)    *A61B 6/00* (2024.01)
*A61B 5/00* (2006.01)    *G06T 11/00* (2006.01)
*A61B 6/02* (2006.01)    *A61B 6/58* (2024.01)

(52) Cooperative Patent Classification (CPC):
**A61B 6/032; A61B 6/5205; A61B 6/582;**
**G06T 11/005;** A61B 5/0035; A61B 5/0064;
A61B 6/027; A61B 6/4405; A61B 6/4441;
A61B 6/4452

(86) International application number:
**PCT/ES2018/070194**

(87) International publication number:
**WO 2018/167348 (20.09.2018 Gazette 2018/38)**

(54) **DEVICE AND METHOD FOR GENERATING TOMOGRAMS**

APPARAT UND METHODE ZUR GENERIERUNG VON TOMOGRAPHIEN

ÉQUIPEMENT ET PROCÉDÉ DE GÉNÉRATION DE TOMOGRAPHIES

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **15.03.2017 ES 201730341**

(43) Date of publication of application:
**25.09.2019 Bulletin 2019/39**

(73) Proprietors:
• **Fundación para la Investigación Biomédica del**
**Hospital Gregorio Marañón**
**28007 Madrid (ES)**
• **Universidad Carlos III de Madrid**
**28919 Leganes (Madrid) (ES)**

(72) Inventors:
• **DESCO MENÉNDEZ, Manuel**
**28007 Madrid (ES)**
• **ABELLA GARCÍA, Mónica**
**28919 Leganes (Madrid) (ES)**

(74) Representative: **Pons**
**Glorieta Rubén Darío 4**
**28010 Madrid (ES)**

(56) References cited:
**DE-A1- 102012 209 422    US-A1- 2008 192 884**
**US-A1- 2011 135 053**

• **SLAGOWSKI JORDAN M ET AL: "Feasibility of**
**CT-based 3D anatomic mapping with a scanning-**
**beam digital x-ray (SBDX) system", PROGRESS**
**IN BIOMEDICAL OPTICS AND IMAGING, SPIE -**
**INTERNATIONAL SOCIETY FOR OPTICAL**
**ENGINEERING, BELLINGHAM, WA, US, vol.**
**9412, 18 March 2015 (2015-03-18), pages 941209 -**
**941209, XP060050961, ISSN: 1605-7422, ISBN:**
**978-1-5106-0027-0, DOI: 10.1117/12.2082052**
• **KERSTIN MÜLLER ET AL: "Fully Automatic Head**
**Motion Correction for Interventional C-arm**
**Systems using Fiducial Markers",**
**PROCEEDINGS OF THE 13TH FULLY THREE-**
**DIMENSIONAL IMAGE RECONSTRUCTION IN**
**RADIOLOGY AND NUCLEAR MEDICINE, 1 July**
**2015 (2015-07-01), pages 534 - 537, XP055491611,**
**Retrieved from the Internet <URL:https://www5.**
**informatik.uni-erlangen.de/Forschung/**
**Publikationen/2015/Mueller15-FAH.pdf>**
**[retrieved on 20180710]**

**(Cont. next page)**

- FAHRIG R ET AL: "Three-dimensional computed tomographic reconstruction using a C-arm mounted XRII: Image-based correction of gantry motion nonidealities", MEDICAL PHYSICS, AIP, MELVILLE, NY, US, vol. 27, no. 1, 1 January 2000 (2000-01-01), pages 30 - 38, XP012010955, ISSN: 0094-2405, DOI: 10.1118/1.598854
- BASTIAN BIER ET AL: "Range Imaging for Motion Compensation in C-Arm Cone-Beam CT of Knees under Weight-Bearing Conditions", JOURNAL OF IMAGING, vol. 4, no. 1, 6 January 2018 (2018-01-06), pages 13, XP055491324, DOI: 10.3390/jimaging4010013
- FOTOUHI JAVAD ET AL: "Can real-time RGBD enhance intraoperative Cone-Beam CT?", INTERNATIONAL JOURNAL OF COMPUTER ASSISTED RADIOLOGY AND SURGERY, SPRINGER, DE, vol. 12, no. 7, 25 March 2017 (2017-03-25), pages 1211 - 1219, XP036276662, ISSN: 1861-6410, [retrieved on 20170325], DOI: 10.1007/S11548-017-1572-Y

## Description

## OBJECT OF THE INVENTION

The present invention falls within the technical field of diagnostic imaging devices. More specifically, an apparatus and a method are described that enable a 3D image to be obtained from a limited number of projections and limited angular range by using X-ray imaging systems with low mechanical precision requirements.

## BACKGROUND OF THE INVENTION

A C-arm imaging system enables 2D planar images that do not provide depth information to be obtained. This type of system comprises at least one X-ray source and one image capture system mounted on an arch-shaped arm. They also comprise a workstation on which the images are displayed, stored and manipulated.

Some advantages of these systems are, for example, that their open design enables placing the C-shaped arm around a patient who is lying down on a bed or on an operating table, and that they are compact and inexpensive compared to other medical imaging systems. Thanks to the open design thereof, this type of system is currently used as a tool to obtain patient images in the operating room.

Two of the main technical problems associated with obtaining 3D images with this type of systems designed for planar images are the following:

- the system can experience mechanical stresses that change the relative positions of the X-ray source and the detector for each projection position along the specific trajectory followed by the X-ray imaging device, without these positions being repeatable for successive acquisitions (low mechanical precision), thus hindering the geometric calibration; and
- in general, they are not designed to take a sufficient number of projections covering a wide angular range around the patient, this being necessary in order to reconstruct tomographic images free from the artifacts typical of the analytical reconstruction of data obtained within a small angular range and with an increased angular step, thus with enough quality so that they are useful for clinical diagnosis (which will be referred to as "clinical (or diagnostic) quality" throughout this document). These methods require projections taken from an angular range of 180 degrees plus the aperture of the emission cone formed between the X-ray source and the detector (called *short scan*) or 360 degrees (called *full scan*).

In the past few years, systems based on specific instrumentation which combines portable X-ray equipment with digital detectors to obtain intraoperative 3D images have been developed (for example the Ziehm Vision FD Vario 3D®, the Siemens Arcadis Orbic Orbital Rotation® or the O-arm by Medtronics®). Nevertheless, these systems maintain strict mechanical precision requirements, generally they only enable isocentric rotations and cover a high angular range.

Likewise, it is known from the state of the art the document DE102012209422A1, which describes an X-ray system comprising an X-ray radiator (source) and a detector, each of them oppositely arranged at a C-arm in order to take images during surgical procedures, as well as a terahertz transmitter and a terahertz receiver for determining the surface of the patient being studied and a control device that utilizes the surface information obtained during reconstruction and/or evaluation processes.

Related to this patent, documents DE102010010192A1 and US2012294504A1 are known, which respectively describe a terahertz transmitter and receiver and a processing unit that shows the irradiated surface and a method for obtaining 3D images free from artifacts in truncation situations, completing the data with information from the surface of the patient, or for determining the relative position of the patient.

The above documents describe an X-ray system with a scanning of the surface for surgical situations wherein the surface is used for the correction of the truncation artifact or for the positioning of the patient, among others. Solutions for avoiding reconstruction artifacts due to a reduced angular range and small number of projections are not described. Furthermore, solutions for the problem of geometric calibration due to lack of mechanical precision (high values of mechanical tolerance that do not ensure the repeatability of the source and detector trajectories) are not described.

The document US2011255765A1 describes an X-ray system with a surface detector for compensating for the metal artifact in dental studies based on the registration of the surface of the teeth with the reconstructed image (eliminating points in the reconstructed image that fall outside the surface). In this document, solutions are not proposed for the problem of image reconstruction from data obtained in a limited angular range and/ a small number of projections, nor for the problem of geometric calibration due to a lack of mechanical precision (high mechanical tolerance values that do not ensure the repeatability of the source and detector trajectory).

Other known patents that make use of a surface scanner are, for example, US 20130034203A1, which describes a method for preparing anatomical simulated images of experimentation animals based on the registration of the surface of the animal with an atlas, and the apparatus for acquiring the data necessary for the surface; US2010010757A1, which describes the use of a surface scanner for gathering part of the data necessary for obtaining attenuation maps; and US2016148398A1, which enables the correction of shifts in the projection data due to geometric deformation and vibration of a C-arm in a short time. In these documents solutions are neither proposed for the problem of image reconstruction from data obtained in limited angular range and a small number of projections, nor for the problem of geometric calibration due to lack of mechanical precision (high mechanical tolerance values that do not ensure the repeatability of the source and detector trajectory).

It is also known document US2011135053, which describes a calibration method for determining the effective center of rotation in not perfectly isocentric 3D rotational C-arm systems and substantially eliminating circular ring artifacts. The geometrical calibration data is obtained by scanning a calibration phantom from a plurality of distinct projection directions and calculating, for each projection direction, the 3D positions of the X-ray tube's focal spot and the X-ray detector's center using a circular regression technique. In this document solutions are neither proposed for the problem of image reconstruction from data obtained in limited angular range and a small number of projections, nor for the problem of geometric calibration due to lack of mechanical precision (high mechanical tolerance values that do not ensure the repeatability of the source and detector trajectory).

## DESCRIPTION OF THE INVENTION

The present invention describes an apparatus and a method for generating tomographies from the data obtained with an X-ray imaging device, which can have low mechanical precision (high mechanical tolerance values that do not ensure the repeatability of the source and detector trajectory), and a surface-scanning device.

The apparatus for generating tomographies of the present invention comprises:

- an X-ray imaging device that in turn comprises:

  - an X-ray source,
  - an X-ray detector,

  where the X-ray source and the X-ray detector can have a joint movement or be moved independently, with the possibility of moving one of them or both with a predetermined trajectory following an isocentric or non-isocentric orbit around the body or body part of which the tomography is to be generated;
- a surface-scanning device configured to obtain data from the surface of the body or body part of which the tomography is to be generated, and that can be the X-ray imaging device itself (in case of complete or non-limited span), or be attached to some part thereof or it can be completely independent from it, including the possibility of it being held and moved manually;
- a data control and processing system configured to execute at least one geometric calibration routine and a routine for generating tomographies that uses the data obtained with the X-ray imaging device combined with the data obtained with the surface-scanning device.

More specifically, the geometric calibration routine is applied periodically, and the routine for generating tomographies comprises the following stages:

- refinement of the geometric calibration of the apparatus using the data obtained with the X-ray imaging device combined with the data obtained with the surface-scanning device. This stage enables the correction of the geometric errors of the relative positions of the source and the detector due to the mechanical tolerances of the system at each projection position along the specific trajectory followed by the X-ray imaging device during the scanning of the body;
- generation of a tomographic image from the projection data obtained with the X-ray imaging device combined with the data obtained with the surface-scanning device that enables a reconstruction of images free from the artifacts typical of the analytical reconstruction from projection data obtained within an angular range less than the one defined for *short scan* and with an increased angular step (greater than one degree).

Throughout the specification, when the terms "source" and "detector" are used, they refer to the X-ray source and to the X-ray detector. Likewise, when reference is made to the body of which the tomography is going to be generated, it refers to a complete body or a part thereof.

It is key for the invention that the projection images obtained with the X-ray imaging device are digital, for which reason in an exemplary embodiment of the invention the detector is a digital flat-panel detector. In another embodiment of the invention, the X-ray imaging device comprises an analog detector and in this case the X-ray imaging device needs to include an image digitalization element.

The X-ray source and the X-ray detector can be moved independently, each of them following a predetermined trajectory, or with a joint movement (for example, in the cases in which the source and the detector are attached to a gantry). The X-ray imaging device can be a CT, C-arm, a digital radiology system with a suspension system that enables the source and the detector to be moved around the sample, etc.

The surface-scanning device can be of the type that uses contact (such as those in the form of pointers with markers followed by optical positioning systems) or of the non-contact type (whether they be are based on a conoscopic holography device, such as the ConoProbe Mark®, on structured light, such as the 3D Artec Eva, or on other types of non-ionizing radiation, such as terahertz imaging, or even ionizing types, such as very low dose X rays) or even the X-ray imaging device itself (in case of complete span).

The method of the present invention comprises initial stages for:

a) periodically performing a geometric calibration of the equipment; and
b) obtaining projection data (images or radiographs) of the body with the X-ray imaging device.

Likewise, the method comprises the following stages:

c) obtaining data from the surface of the body with the surface-scanning device;
d) executing the routine for generating tomographies that uses the projection data obtained with the X-ray imaging device combined with the data obtained with the surface-scanning device and comprises at least the following substages:

d1) obtaining a 3D mask of the body from the data obtained with the surface-scanning device;
d2) making a preliminary reconstruction of the projection images obtained by the X-ray imaging device by using the geometric calibration periodically obtained in stage a) and a conventional reconstruction method;
d3) making a 3D registration of the preliminary reconstruction obtained in substage d2) with the 3D mask of the body obtained in substage d1) from the data obtained with the surface-scanning device;
d4) refining the initial geometric calibration of the apparatus obtained periodically in stage a) by using the projection data (images/radiographs) obtained with the X-ray imaging device in stage b) combined with projections of the 3D mask of the body registered in substage d3) simulated with the initial geometric calibration of the apparatus periodically obtained in stage a). This substage enables the correction of the geometric errors of the relative positions of the source and the detector at each projection position along the specific trajectory followed by the X-ray imaging device during the scanning of the body due to the mechanical tolerances thereof;

d5) generation of a tomographic image from the projection data obtained with the X-ray imaging device in stage b) combined with the 3D mask of the body registered in substage d3) that enables a tomographic image free from the artifacts typical of the analytical reconstruction to be reconstructed from projection data obtained within an angular range less than the one defined for short scan and with an increased angular step (greater than one degree).

Thanks to the combination of the apparatus and the method described, with very limited data (small number of projections and/or projections obtained within a small angular range of movement of the source-detector assembly), and/or data taken in a limited angular range of movement of the source and of the detector, and/or high mechanical tolerance to imprecisions in the source and detector positioning (low mechanical precision), results are obtained that are equivalent to those obtained with traditional CT scanners or other types of specific 3D X-ray systems, in which the source-detector assembly rotates 180 degrees plus the aperture of the emission cone made between the source and the detector (called *short scan*) or 360 degrees (called *full scan*).

One of the advantages of the present invention is that the apparatus has a lesser requirement for angular range in the rotation of the source-detector assembly. This is especially advantageous in the cases in which is it not possible to completely encircle the patient, for example in the ICU or during surgery, in which the patient is kept connected to equipment for monitoring vital signs, among others, and where portable C-arm systems are usually used which enable 2D images to be acquired but are not designed to obtain 3D images.

Another associated advantage is that it enables increasing the acquisition speed of tomography, as it can obtain images of clinical quality (suitable for clinical diagnosis) with a smaller angular range of rotation of the source-detector assembly and a fewer number of projections, facilitating performing dynamic studies. When referring to images of clinical quality we mean images which are free from the reconstruction artifacts typical of the analytical reconstruction of data acquired within an angular range less than the one defined for *short scan* and with an increased angular step (greater than one degree).

Likewise, the apparatus and the method enable lower doses of radiation for the patient as it obtains a tomography free from the reconstruction artifacts typical of the analytical reconstruction of data obtained within an angular range less than the one defined for short scan and with an increased angular step (greater than one degree).

Additionally, the apparatus and the method enable the use of pre-existing 2D radiology systems to be extended by the incorporation of tomographic capacities in them. Furthermore, the apparatus for generating tomographies can be portable if the X-ray imaging device is a C-arm, for example. This is especially advantageous in cases in which there are mobility difficulties with the patient, for example in the ICU or during surgery.

Finally, the present invention demands lower mechanical precision requirements in the source and detector positioning, since the stage of refining the geometric calibration of the apparatus of the proposed method for generating tomographies enables variations of the trajectory followed by the X-ray imaging device (source and detector) in successive acquisitions to be corrected. This enables reducing costs, an especially important advantage in veterinary applications or in developing countries with few resources.

In the equipment and methods known in the state of the art, it is necessary to perform a periodic calibration that is valid for subsequent acquisitions given that they have a mechanical precision that is high enough to ensure the repeatability of the trajectory of the source and detector, which prevents the appearance of systematic errors in the images (artifacts).

In the state of the art, there is no solution to the problem of geometric calibration in the cases of low mechanical precision of the X-ray imaging device. Likewise, state-of-the-art systems are not described that enable the elimination of reconstruction artifacts due to a reduced angular range and small number of projections.

## DESCRIPTION OF THE DRAWINGS

As a complement to the description provided herein and with the purpose of helping to make the characteristics of the invention more readily understandable, in accordance with preferred practical embodiments thereof, said description is accompanied by a set of drawings which, by way of illustration and not limitation, represent the following:

Figure 1a shows a view of an embodiment of the apparatus for generating tomographies.
Figure 1b shows a view of another embodiment of the apparatus for generating tomographies with the surface-scanning device arranged in a different position.
Figure 2 shows a flowchart of the method for generating tomographies.
Figure 3 shows graphs with a result obtained with the stage for refining the geometric calibration of the apparatus for two geometric parameters (horizontal shift and skew (rotation) of the detector).
Figure 4a shows sagittal, coronal and axial cross sections of the 3D reconstruction of a body generated from the projection data obtained with the X-ray imaging device by means of a method of the state of the art, before performing the refinement of the geometric calibration of the apparatus.
Figure 4b shows sagittal, coronal and axial cross sections of the 3D reconstruction of a body generated by means of the same method of the state of the art as Figure 4a, after having performed the refinement of the geometric calibration of the apparatus.
Figure 4c shows sagittal, coronal and axial cross sections of the 3D reconstruction of a body generated by means of the complete method for generating tomographies of the present invention.

## PREFERRED EMBODIMENT OF THE INVENTION

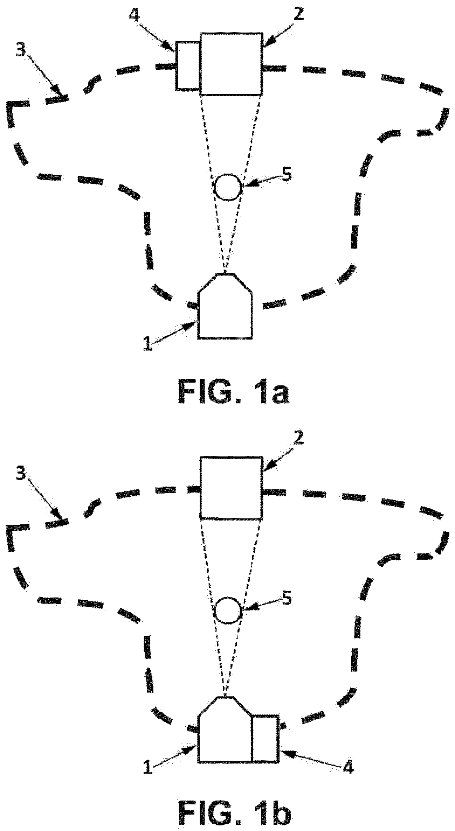

What follows is a description, with the help of Figures 1 to 4, of exemplary embodiments of the apparatus and the method for generating tomographies of the present invention. Likewise, the resulting tomographies are shown (views of sagittal, coronal and axial cross sections of 3D reconstructions of a body) generated from the data obtained with an X-ray imaging device by means of a method of the state of the art (FDK) and by means of the different stages of the method for generating tomographies of the present invention.

A first object of the invention is the apparatus for generating tomographies. In Figure 1, an exemplary embodiment is shown of said apparatus comprising at least one X-ray imaging device, a surface-scanning device and a data control and processing system.

The X-ray imaging device comprises an X-ray source (1) and an X-ray detector (2); one of these elements or both can move.

The X-ray source (1) and the X-ray detector (2) are facing each other in the imaging device, and the body (5) of which the tomography is to be generated is placed between them. The X-ray source (1) or the X-ray detector (2) or both are moved following a predefined orbit (3) around the body (5) that can be isocentric or not isocentric. In Figures 1a and 1b, exemplary embodiments are shown in which the orbit (3) is not circular and the X-ray source (1) and the X-ray detector (2) are moved independently from each other.

The movement of the X-ray source (1) or of the X-ray detector (2) or of both is what enables 2D images (radiographs) of the body (5) to be obtained from different views or projection angles. As described previously, the apparatus and the method herein proposed enable tomographies of clinical quality (suitable for clinical diagnosis) to be

6

obtained even in cases in which the movement of the source-detector assembly is limited to a small number of views or a limited angular range.

The apparatus also comprises a surface-scanning device (4) configured to obtain data from the surface of the body (5) of which the tomography is to be generated. Said surface-scanning device (4) can be arranged in different positions in the apparatus (attached to the X-ray source, to the X-ray detector or be completely independent, including the possibility of it being held and moved manually) or be the X-ray imaging device itself (in case of complete span), without this limiting the correct acquisition of the surface.

Likewise, the apparatus comprises a data control and processing system configured to execute at least a geometric calibration routine and a routine for generating tomographies. Preferably, the routine for generating tomographies that is executed in the data control and processing system comprises the following stages:

- a stage for refining the geometric calibration of the apparatus performed periodically by using the projection data obtained with the X-ray imaging device (1, 2) combined with the data obtained with the surface-scanning device (4). This stage enables the correction of the geometric errors of the relative positions of the source and the detector at each projection position along the specific trajectory followed by the X-ray imaging device (1, 2) during the scanning of the body (5) due to the mechanical tolerances thereof;
- a stage for generating a tomographic image from the projection data obtained with the X-ray imaging device (1, 2) combined with the data obtained with the surface-scanning device (4) that enables the reconstruction of images free from the reconstruction artifacts typical of the analytical reconstruction of data obtained within an angular range less than the one defined for *short scan* and with an increased angular step (greater than one degree).

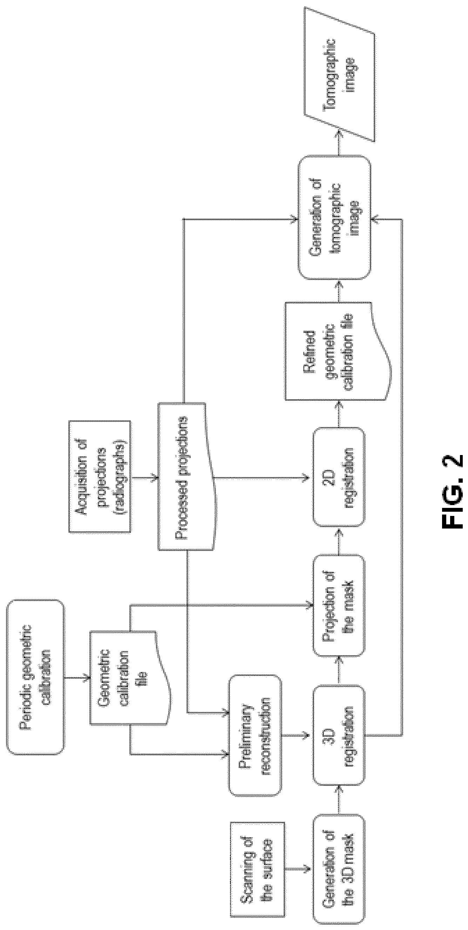

A method for generating tomographies with the apparatus for generating tomographies is also an object of the invention. The tomographies are generated from the data acquired with the X-ray imaging device and the surface-scanning device. In Figure 2, the different stages of the method are shown.

The initial stages of the method are:

a) performing a geometric calibration of the apparatus;
b) obtaining projection images (radiographs) with the X-ray imaging device (1, 2).

Stage a), geometric calibration of the apparatus, enables the position of the X-ray source and of the X-ray detector for each view (projection position) to be determined. This stage is carried out periodically in the apparatus.

In an exemplary embodiment, the method could also comprise a pre-processing stage for the images obtained with the X-ray imaging device, in case the detector obtains images as raw (unprocessed) data.

Subsequently, the method comprises performing the following stages:

c) obtaining data from the surface of the body (5) with the surface-scanning device (4);
d) executing the routine for generating tomographies that uses the projection data obtained with the X-ray imaging device (1, 2) combined with the data obtained with the surface-scanning device (4) and comprises at least the following substages:

d1) obtaining a 3D mask of the body (5) of which a tomography is to be generated from the surface data obtained with the surface-scanning device (4);
d2) making a preliminary reconstruction of the projection images obtained with the X-ray imaging device (1, 2) by using the geometric calibration periodically obtained in stage a) and a conventional reconstruction method.
d3) performing a 3D registration of the preliminary reconstruction obtained in stage d2) with the 3D mask of the body obtained in substage d1) from the data obtained with the surface-scanning device;
d4) refining the initial geometric calibration of the apparatus performed previously in stage a) by using the projection data obtained with the X-ray imaging device (1, 2) in stage b) combined with projections of the 3D mask of the body registered in substage d3) simulated with the initial geometric calibration of the device. This substage enables the correction of the geometric errors of the relative positions of the source and the detector at each projection position along the specific trajectory followed by the X-ray imaging device (1, 2) during the scanning of the body (5), due to the mechanical tolerances thereof;
d5) generation of a tomographic image from the projection data obtained with the X-ray imaging device (1, 2) combined with the 3D mask registered in substage d3) which enables images of clinical quality to be reconstructed with data obtained within an angular range less than the one defined for *short scan* and with an increased angular step (greater than one degree).

For the source and detector positions obtained to be valid for subsequent acquisitions, it is necessary to ensure submillimetric mechanical precision in linear shifts of the source and the detector and below the degree in rotation steps of the source and the detector. If the mechanical precision does not ensure a repeatability of source and detector positioning within these values, the geometric parameters must be refined so that they represent the real positions of the source and the X-ray detector during the actual acquisition of the projection images with the X-ray imaging device. Thus, the stage of refining the geometric calibration of the apparatus (substage d4), is essential in the described method.

Substage d4) for refining the geometric calibration of the apparatus preferably comprises the following steps:

i) simulating the projection of the 3D mask registered in substage d3) with the initial geometric calibration of the apparatus periodically obtained in stage a); and

ii) performing a registration of the projections of the registered mask, obtained in step i) with the projection images obtained with the X-ray imaging device in stage b) projection by projection;

iii) refining the parameters of the geometric calibration periodically obtained in stage a) with the registration values obtained in step ii) in order to obtain a refined calibration file.

In an exemplary embodiment, substage d2) is carried out by means of a conventional FDK reconstruction method with the geometric calibration of the apparatus obtained (periodically) in stage a).

In Figure 2, all the steps of the key substages of the present invention are shown, which are the substage for refining the geometric calibration of the apparatus, previously described (substage d4)), and for generating a tomographic image (substage d5)), both using the projection data obtained with the X-ray imaging device combined with the data obtained with the surface-scanning device. These substages of the method are the ones that enable a tomographic image of clinical quality (images free from the reconstruction artifacts typical of the analytical reconstruction of data obtained within an angular range less than the one defined for *short scan* and with an increased angular step (greater than one degree)) to be generated despite using a small number of projections and projections obtained within a limited angular range of movement of the source-detector assembly with the apparatus of the invention with low mechanical precision requirements.

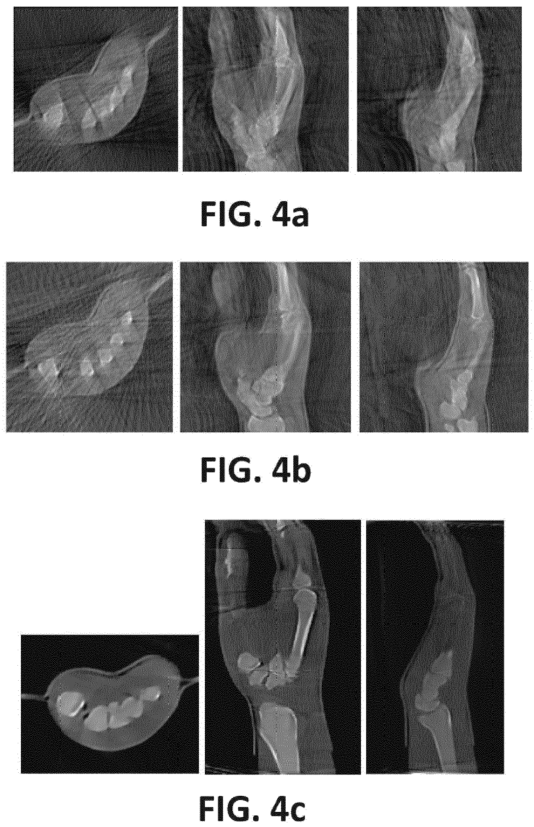

In an example of tomography generation using an apparatus like the one shown in Figure 1, in order to achieve the different projection images (planar images) with the X-ray imaging device, the X-ray source (1) and the X-ray detector (2) are rotated together following an almost circular orbit (3) around the body (5) since in this example the source and the detector are attached to a gantry. In this exemplary embodiment, in order to obtain the images of Figures 4a-c, 42 projections distributed uniformly in an angular range of 120 degrees are acquired.

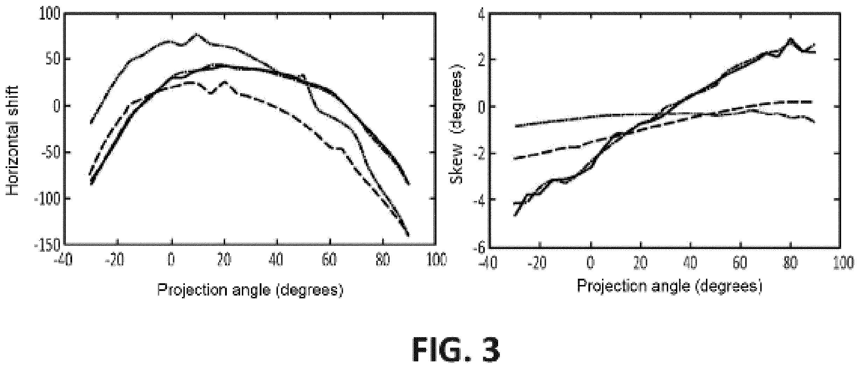

Figure 3 shows the result of the first key stage of the method for generating tomographies of the invention, which is the stage of refining the geometric calibration of the apparatus using the surface data obtained with the surface-scanning device (4). The figure shows the values of two of the main geometric parameters, horizontal shift and skew (rotation) of the detector (2), for two different geometric calibrations before and after the calibration refinement.

Said Figure 3 shows the difference in the geometric parameters for the same X-ray imaging device obtained by performing the geometric calibration at two different times (lack of mechanical precision in the X-ray imaging device) and the need of substage d4) of the method for generating tomographies of the invention, which is the stage of refining the geometric calibration of the apparatus using the projection data obtained with the X-ray imaging device itself combined with the registered 3D mask generated with the data obtained with the surface-scanning device. In one of the graphs of Figure 3, the horizontal shift of the detector in millimeters corresponding to each projection angle in degrees is shown. In the other graph, the skew (rotation) is represented in degrees for each projection angle, also in degrees. In both cases, the dashed line represents a first calibration and the line with a dash-dot-dash pattern represents that same first calibration after refinement. Likewise, the line with a dash-dot-dot-dash pattern represents a second calibration and the solid line represents that same second calibration, after refinement. After the refinement of the geometric calibration, the parameters coincide with the actual position of the X-ray imaging device when the images of the body are acquired.

Figure 4a shows the results obtained with the 2D X-ray imaging device by means of a state-of-the-art method, before performing the calibration refinement. Figure 4b shows the results obtained with the same device and the same state-of-the-art method as Figure 4a after performing the stage of refining the geometric calibration of the apparatus using the projection images combined with the surface data obtained previously.

Figure 4c shows an example of good quality tomography obtained with the apparatus and the method of the invention. As seen in said figure, the reconstructed image has good quality despite having been generated from very limited data and without having ensured the mechanical precision that enables the repeatability of the source and detector trajectory.

In this case, a tomography of clinical quality (images free from the reconstruction artifacts typical of the analytical reconstruction of data obtained within an angular range less than the one defined for short scan and with an increased angular step (greater than one degree)) has been obtained with only 42 projections compared to the more than 400 projections usually performed in state-of-the-art CT scanners. Furthermore, the angular range of the movements of the source-detector assembly was 120 degrees, compared to the usual 360 degrees for the acquisitions with state-of-the-art systems with *full scan,* or the 180 degrees plus the aperture of the emission cone formed between the X-ray source and the detector, necessary in a short acquisition (*short scan*).

In this exemplary embodiment, the reconstruction method used was based on the minimization of the Total Variation function (TV) subjected to the restrictions: (a) data fidelity and (b) that the result is contained within the 3D mask generated from the data obtained with the surface-scanning device. In this case, the following formulation was used:

$$\min \left\| \nabla \left( \mathrm{u} \right) \right\|_1 \ \mathrm{s.t.} u \eth \, \Omega, \left\| \mathrm{Au} - \mathrm{f} \right\|_2^2 < \sigma^2$$

$$\nabla \left( \mathrm{u} \right)_1 = \sqrt{ \left( \nabla_x u \right)^2 + \left( \nabla_y u \right)^2 }$$

where u is the reconstructed image, and $\nabla_x$ and $\nabla_y$ are the gradients of *u* in the directions x and *y, A* is the system matrix, *f* is the data acquired by the X-ray imaging device, $\sigma^2$ is the noise in these data and $\Omega$ is the registered 3D mask generated from the data acquired with the surface-scanning device. The resolution of the minimization problem was done using the Split Bregman formulation.

## Claims

1. Apparatus for generating tomographies comprising:

   - an X-ray imaging device that in turn comprises:

     - an X-ray source (1),
     - an X-ray detector (2),

       and the X-ray source (1) and the X-ray detector (2) are facing each other in order to receive between them a body (5) of which the tomography will be generated, obtaining projection data within an angular range less than the one defined for *short scan* and with an increased angular step, greater than one degree; and the X-ray source (1) and the X-ray detector (2) have a joint movement or are moved independently, with a predetermined trajectory following an isocentric or non-isocentric orbit (3);

   - a surface-scanning device (4) configured to obtain data from the surface of the body (5),
   - a data control and processing system configured to execute:

     - a routine for the geometric calibration of the apparatus that is applied periodically, and

       - a routine for generating tomographies that uses the projection data obtained with the X-ray imaging device (1, 2) combined with the data obtained with the surface-scanning device (4) and performs at least the following substages: generation of a 3D mask (5) of the body from the data obtained with the surface-scanning device (4); preliminary reconstruction of the projection images obtained with the X-ray imaging device (1, 2) by using the geometric calibration periodically obtained; 3D registration of the preliminary reconstruction obtained with the 3D mask of the body (5) from the data obtained with the surface-scanning device (4); refinement of the geometric calibration of the apparatus using the projection data obtained with the X-ray imaging device (1, 2) combined with the 3D mask of the body (5) registered that enables the correction of the geometric errors of the relative positions of the source and the detector at each projection position along the specific trajectory followed by the X-ray imaging device (1, 2) during the scanning of the body (5) due to the mechanical tolerances thereof; and generation of a tomographic image from the projection data obtained with the X-ray imaging device (1, 2) combined with the 3D mask of the body (5) registered, thus reconstructing images suitable for clinical diagnosis, by elimination of reconstruction artifacts from data obtained within an angular range less than the one defined for *short scan* and with an increased angular step, greater than one degree.

2. The apparatus for generating tomographies according to claim 1, **characterized in that** the surface-scanning device (4) is a contact scanning device.

3. The apparatus for generating tomographies according to claim 1, **characterized in that** the surface-scanning device (4) is a non-contact scanning device.

4. The apparatus for generating tomographies according to claim 1, **characterized in that** the surface-scanning device (4) is attached to a part of the X-ray imaging device (1, 2).

5. The apparatus for generating tomographies according to claim 1, **characterized in that** the surface-scanning device (4) is completely independent from the X-ray imaging device (1, 2), including the possibility of it being held and moved manually.

6. A method for generating tomographies with the apparatus for generating tomographies of claims 1 to 5 comprising the stages of:

   a) periodically performing the geometric calibration of the equipment;
   b) obtaining projection images, with the X-ray imaging device;

   and is **characterized in that** it subsequently comprises the following stages:

   c) obtaining data from the surface of a body (5) with the surface-scanning device (4);
   d) executing the routine for generating tomographies that uses the projection images obtained with the X-ray imaging device (1, 2) combined with the data obtained with the surface-scanning device (4) and comprises at least the following stages:

   d1) generation of a 3D mask (5) of the body from the data obtained with the surface-scanning device (4);
   d2) preliminary reconstruction of the projection images obtained with the X-ray imaging device (1, 2) by using the geometric calibration periodically obtained in stage a);
   d3) 3D registration of the preliminary reconstruction obtained in substage d2) with the 3D mask of the body (5) obtained in substage d1) from the data obtained with the surface-scanning device (4);
   d4) refinement of the geometric calibration of the apparatus using the projection data obtained with the X-ray imaging device (1, 2) in stage b) combined with the 3D mask of the body (5) registered in substage d3) that enables the correction of the geometric errors of the relative positions of the source and the detector at each projection position along the specific trajectory followed by the X-ray imaging device (1, 2) during the scanning of the body (5) due to the mechanical tolerances thereof,
   d5) generation of a tomographic image from the projection data obtained with the X-ray imaging device (1, 2) in stage b) combined with the 3D mask of the body (5) registered in substage d3), thus obtaining images suitable for clinical diagnosis from data obtained within an angular range less than the one defined for *short scan* and with an increased angular step, greater than one degree.

7. The method for generating tomographies according to claim 6, **characterized in that** substage d4), refinement of the geometric calibration of the apparatus, comprises the following steps:

   i) simulating the projections of the 3D mask of the body registered in substep d3) by using the initial geometric calibration of the apparatus obtained periodically in stage a);
   ii) performing a registration, projection by projection, of the simulated projections of the registered 3D mask obtained in step i), with the projection images obtained by the X-ray imaging device in stage b); and
   iii) refining the geometric calibration of the apparatus periodically obtained in stage a) with the registration values obtained in step ii) in order to obtain a refined calibration file with the relative positions of the source and the detector for each projection position in the specific trajectory followed by the X-ray imaging device during the scanning of the body.

**Patentansprüche**

1. Gerät zur Erzeugung von Tomografien, das Folgendes umfasst:

   - eine Röntgenbildgebungsvorrichtung, die ihrerseits Folgendes umfasst:

      - eine Röntgenquelle (1),

- einen Röntgendetektor (2),

und die Röntgenquelle (1) und der Röntgendetektor (2) zueinander zeigen, um zwischen sich einen Körper (5), von dem die Tomografie erzeugt wird, aufzunehmen, wobei Projektionsdaten in einem Winkelbereich, der kleiner ist als der für einen *Kurzscan* definierte, und mit einem vergrößerten Winkelschritt von mehr als einem Grad gewonnen werden; und die Röntgenquelle (1) und der Röntgendetektor (2) eine gemeinsame Bewegung aufweisen oder unabhängig voneinander bewegt werden, mit einer zuvor festgelegten Bahnkurve, die einem isozentrischen oder nicht isozentrischen Umlauf folgt (3);

- eine Oberflächenabtastvorrichtung (4), die so konfiguriert ist, dass sie Daten von der Oberfläche des Körpers (5) gewinnt,
- ein Datensteuerungs- und -verarbeitungssystem, das konfiguriert ist, um Folgendes auszuführen:

- eine Routine für die geometrische Kalibrierung des Geräts, die periodisch angewendet wird, und

- eine Routine für das Erzeugen von Tomografien, die die mit der Röntgenbildgebungsvorrichtung (1, 2) gewonnenen Projektionsdaten in Kombination mit den mit der Oberflächenabtastvorrichtung (4) gewonnenen Daten verwendet und mindestens die folgenden Teilstufen durchführt: Erzeugung einer 3D-Maske (5) des Körpers aus den mit der Oberflächenabtastvorrichtung (4) gewonnenen Daten; vorläufige Rekonstruktion der mit der Röntgenbildgebungsvorrichtung (1, 2) gewonnenen Projektionsbilder unter Verwendung der periodisch gewonnenen geometrischen Kalibrierung; 3D-Registrierung der vorläufigen Rekonstruktion, die mit der 3D-Maske des Körpers (5) aus den mit der Oberflächenabtastvorrichtung (4) gewonnenen Daten gewonnen wurde; Verfeinerung der geometrischen Kalibrierung des Geräts unter Verwendung der mit der Röntgenbildgebungsvorrichtung (1, 2) gewonnenen Projektionsdaten in Kombination mit der registrierten 3D-Maske des Körpers (5), die die Korrektur der geometrischen Fehler der relativen Positionen der Quelle und des Detektors an jeder Projektionsposition entlang der spezifischen Bahnkurve, der die Röntgenbildgebungsvorrichtung (1, 2) während der Abtastung des Körpers (5) aufgrund ihrer mechanischen Toleranzen folgt, ermöglicht, und Erzeugung eines tomografischen Bildes aus den mit der Röntgenbildgebungsvorrichtung (1, 2) gewonnenen Projektionsdaten in Kombination mit der registrierten 3D-Maske des Körpers (5), wodurch durch Beseitigung von Rekonstruktionsartefakten aus Daten, die in einem Winkelbereich, der kleiner ist als der für einen *Kurzscan* definierte, und mit einem vergrößerten Winkelschritt von mehr als einem Grad gewonnen wurden, für die klinische Diagnose geeignete Bilder rekonstruiert werden.

2. Gerät zur Erzeugung von Tomografien nach Anspruch 1, **dadurch gekennzeichnet, dass** die Oberflächenabtastvorrichtung (4) eine Kontaktabtastvorrichtung ist.

3. Gerät zur Erzeugung von Tomografien nach Anspruch 1, **dadurch gekennzeichnet, dass** die Oberflächenabtastvorrichtung (4) eine berührungslose Abtastvorrichtung ist.

4. Gerät zur Erzeugung von Tomografien nach Anspruch 1, **dadurch gekennzeichnet, dass** die Oberflächenabtastvorrichtung (4) an einem Teil der Röntgenbildgebungsvorrichtung (1, 2) angebracht ist.

5. Gerät zur Erzeugung von Tomografien nach Anspruch 1, **dadurch gekennzeichnet, dass** die Oberflächenabtastvorrichtung (4) völlig unabhängig von der Röntgenbildgebungsvorrichtung (1, 2) ist, einschließlich der Möglichkeit, dass sie manuell gehalten und bewegt wird.

6. Verfahren zur Erzeugung von Tomografien mit dem Gerät zur Erzeugung von Tomografien nach einem der Ansprüche 1 bis 5, das die folgenden Stufen umfasst:

a) regelmäßiges Durchführen der geometrischen Kalibrierung der Einrichtung;
b) Gewinnen von Projektionsbildern mit der Röntgenbildgebungsvorrichtung;

und **dadurch gekennzeichnet ist, dass** es anschließend die folgenden Stufen umfasst:

c) Gewinnen von Daten von der Oberfläche eines Körpers (5) mit der Oberflächenabtastvorrichtung (4);

d) Ausführen der Routine zur Erzeugung von Tomografien, die die mit der Röntgenbildgebungsvorrichtung (1, 2) gewonnenen Projektionsbilder in Kombination mit den mit der Oberflächenabtastvorrichtung (4) gewonnenen Daten verwendet und mindestens die folgenden Stufen umfasst:

d1) Erzeugen einer 3D-Maske (5) des Körpers aus den mit der Oberflächenabtastvorrichtung (4) gewonnenen Daten;

d2) vorläufiges Rekonstruieren der mit der Röntgenbildgebungsvorrichtung (1, 2) gewonnenen Projektionsbilder unter Verwendung der in Stufe a) periodisch gewonnenen geometrischen Kalibrierung;

d3) 3D-Registrieren der in Teilstufe d2) gewonnenen vorläufigen Rekonstruktion mit der 3D-Maske des Körpers (5), die in Teilstufe d1) aus den mit der Oberflächenabtastvorrichtung (4) gewonnenen Daten gewonnen wurde;

d4) Verfeinern der geometrischen Kalibrierung des Geräts unter Verwendung der mit der Röntgenbildgebungsvorrichtung (1, 2) in Stufe b) gewonnenen Projektionsdaten in Kombination mit der in Teilstufe d3) registrierten 3D-Maske des Körpers (5), das die Korrektur der geometrischen Fehler der relativen Positionen der Quelle und des Detektors an jeder Projektionsposition entlang der spezifischen Bahnkurve, der die Röntgenbildgebungsvorrichtung (1, 2) während der Abtastung des Körpers (5) aufgrund ihrer mechanischen Toleranzen folgt, ermöglicht,

d5) Erzeugen eines tomografischen Bildes aus den mit der Röntgenbildgebungsvorrichtung (1, 2) in Stufe b) gewonnenen Projektionsdaten in Kombination mit der in Teilstufe d3) registrierten 3D-Maske des Körpers (5), wodurch für die klinische Diagnose geeignete Bilder aus Daten gewonnen werden, die in einem Winkelbereich, der kleiner ist als der für einen *Kurzscan* definierte, und mit einem vergrößerten Winkelschritt von mehr als einem Grad gewonnen wurden.

7. Verfahren zur Erzeugung von Tomografien nach Anspruch 6, **dadurch gekennzeichnet, dass** Teilstufe d4), das Verfeinern der geometrischen Kalibrierung des Geräts, die folgenden Schritte umfasst:

i) Simulieren der Projektionen der in Teilstufe d3) registrierten 3D-Maske des Körpers unter Verwendung der anfänglichen geometrischen Kalibrierung des Geräts, die periodisch in Stufe a) gewonnen wird;

ii) Durchführen einer Registrierung, Projektion für Projektion, der simulierten Projektionen der in Schritt i) gewonnenen registrierten 3D-Maske mit den von der Röntgenbildgebungsvorrichtung in Stufe b) gewonnenen Projektionsbildern; und

iii) Verfeinern der in Stufe a) periodisch gewonnenen geometrischen Kalibrierung des Geräts mit den in Schritt ii) gewonnenen Registrierungswerten, um eine verfeinerte Kalibrierungsdatei mit den relativen Positionen der Quelle und des Detektors für jede Projektionsposition in der spezifischen Bahnkurve, der die Röntgenbildgebungsvorrichtung während der Abtastung des Körpers folgt, zu gewinnen.

## Revendications

1. Appareil de génération de tomographies comprenant :

- un dispositif d'imagerie à rayons X qui comprend à son tour :

- une source de rayons X (1),
- un détecteur de rayons X (2),

et la source de rayons X (1) et le détecteur de rayons X (2) se font face afin de recevoir entre eux un corps (5) dont la tomographie sera générée, obtenant des données de projection dans une plage angulaire inférieure à celle définie pour le *balayage court* et avec un pas angulaire accru, supérieur à un degré ;
et la source de rayons X (1) et le détecteur de rayons X (2) ont un mouvement conjoint ou sont mus indépendamment,
avec une trajectoire prédéterminée suivant une orbite isocentrique ou non isocentrique (3) ;

- un dispositif de balayage de surface (4) configuré pour obtenir des données de la surface du corps (5),
- un système de contrôle et de traitement des données configuré pour exécuter :

- une routine d'étalonnage géométrique de l'appareil qui est appliquée périodiquement, et

- une routine de génération de tomographies qui utilise les données de projection obtenues avec le dispositif d'imagerie à rayons X (1, 2) combinées aux données obtenues avec le dispositif de balayage de surface (4) et effectue au moins les sous-étapes suivantes : génération d'un masque 3D (5) du corps à partir des données obtenues avec le dispositif de balayage de surface (4) ; reconstruction préliminaire des images de projection obtenues avec le dispositif d'imagerie à rayons X (1, 2) à l'aide de l'étalonnage géométrique obtenu périodiquement ; enregistrement 3D de la reconstruction préliminaire obtenue avec le masque 3D du corps (5) à partir des données obtenues avec le dispositif de balayage de surface (4) ; affinement de l'étalonnage géométrique de l'appareil à l'aide des données de projection obtenues avec le dispositif d'imagerie à rayons X (1, 2) combiné au masque 3D du corps (5) enregistré, qui permet la correction des erreurs géométriques des positions relatives de la source et du détecteur à chaque position de projection le long de la trajectoire spécifique suivie par le dispositif d'imagerie à rayons X (1, 2) pendant le balayage du corps (5) en raison des tolérances mécaniques de ce dernier ; et génération d'une image tomographique à partir des données de projection obtenues avec le dispositif d'imagerie à rayons X (1, 2) combinées avec le masque 3D du corps (5) enregistré, reconstruisant ainsi des images adaptées au diagnostic clinique, par l'élimination d'artefacts de reconstruction à partir de données obtenues dans une plage angulaire inférieure à celle définie pour le *balayage court* et avec un pas angulaire accru, supérieur à un degré.

2. Appareil de génération de tomographies selon la revendication 1, **caractérisé en ce que** le dispositif de balayage de surface (4) est un dispositif de balayage par contact.

3. Appareil de génération de tomographies selon la revendication 1, **caractérisé en ce que** le dispositif de balayage de surface (4) est un dispositif de balayage sans-contact.

4. Appareil de génération de tomographies selon la revendication 1, **caractérisé en ce que** le dispositif de balayage de surface (4) est fixé à une partie du dispositif d'imagerie à rayons X (1, 2).

5. Appareil de génération de tomographies selon la revendication 1, **caractérisé en ce que** le dispositif de balayage de surface (4) est complètement indépendant du dispositif d'imagerie à rayons X (1, 2), y compris la possibilité d'être tenu et mû manuellement.

6. Procédé de génération de tomographies avec l'appareil de génération de tomographies selon les revendications 1 à 5 comprenant les étapes consistant à :

a) effectuer périodiquement l'étalonnage géométrique de l'équipement ;
b) obtenir des images de projection, avec le dispositif d'imagerie à rayons X ;

et est **caractérisé en ce qu'**il comprend ensuite les étapes suivantes :

c) obtenir des données sur la surface d'un corps (5) à l'aide du dispositif de balayage de surface (4) ;
d) exécuter la routine de génération de tomographies qui utilise les images de projection obtenues avec le dispositif d'imagerie à rayons X (1, 2) combinées avec les données obtenues avec le dispositif de balayage de surface (4) et comprend au moins les étapes suivantes :

d1) génération d'un masque 3D (5) du corps à partir des données obtenues avec le dispositif de balayage de surface (4) ;
d2) reconstruction préliminaire des images de projection obtenues avec le dispositif d'imagerie à rayons X (1, 2) à l'aide de l'étalonnage géométrique obtenu périodiquement à l'étape a) ;
d3) enregistrement 3D de la reconstruction préliminaire obtenue à l'étape d2) avec le masque 3D du corps (5) obtenu à l'étape d1) à partir des données obtenues avec le dispositif de balayage de surface (4) ;
d4) affinement de l'étalonnage géométrique de l'appareil à l'aide des données de projection obtenues avec le dispositif d'imagerie à rayons X (1, 2) à l'étape b) combinées au masque 3D du corps (5) enregistré à l'étape d3) qui permet la correction des erreurs géométriques des positions relatives de la source et du détecteur à chaque position de projection le long de la trajectoire spécifique suivie par le dispositif d'imagerie à rayons X (1, 2) pendant le balayage du corps (5) en raison des tolérances mécaniques de ce dernier,
d5) génération d'une image tomographique à partir des données de projection obtenues avec le dispositif d'imagerie à rayons X (1, 2) à l'étape b), combinées avec le masque 3D du corps (5) enregistré à l'étape d3), obtenant ainsi des images adaptées au diagnostic clinique à partir de données obtenues dans une plage angulaire inférieure à celle définie pour le *balayage court* et avec un pas angulaire accru, supérieur à un degré.

7. Procédé de génération de tomographies selon la revendication 6, **caractérisé en ce que** ce sous-étape d4), l'affinement de l'étalonnage géométrique de l'appareil, comprend les étapes suivantes :

i) simuler les projections du masque 3D du corps enregistré à la sous-étape d3) à l'aide de l'étalonnage géométrique initial de l'appareil obtenu périodiquement à l'étape a) ;

ii) effectuer un enregistrement, projection par projection, des projections simulées du masque 3D enregistré obtenu à l'étape i), avec les images de projection obtenues par le dispositif d'imagerie à rayons X à l'étape b) ; et

iii) affiner l'étalonnage géométrique de l'appareil obtenu périodiquement à l'étape a) avec les valeurs d'enregistrement obtenues à l'étape ii) afin d'obtenir un fichier d'étalonnage affiné avec les positions relatives de la source et du détecteur pour chaque position de projection dans la trajectoire spécifique suivie par le dispositif d'imagerie à rayons X pendant le balayage du corps.

FIG. 1a

FIG. 1b

FIG. 2

FIG. 3

**FIG. 4a**

**FIG. 4b**

**FIG. 4c**

**EP 3 542 722 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 102012209422 A1
- DE 102010010192 A1
- US 2012294504 A1
- US 2011255765 A1
- US 20130034203 A1
- US 2010010757 A1
- US 2016148398 A1
- US 2011135053 A